# FASCICULE DE BREVET EUROPEEN

(11) **EP 2 297 176 B1**
(45) Date de publication et mention de la délivrance du brevet: **07.12.2011**
(21) Numéro de dépôt: 09761895.3
(22) Date de dépôt: 14.05.2009
(51) Int. Cl.: C07H 21/00, C07H 21/02, A61K 31/7088

(54) **NOUVEAU SYSTEME DE TRANSFERT D'ACIDE NUCLEIQUE**
NEUES NUKLEINSÄURETRANSFERSYSTEM
NOVEL NUCLEIC ACID TRANSFER SYSTEM

(30) Priorité: 16.05.2008 FR 0853205
(43) Date de publication de la demande: 23.03.2011
(73) Titulaire: Centre National de la Recherche Scientifique, 75016 Paris Cedex 16 (FR); Universite Paris-Sud, 91400 Orsay (FR)
(72) Inventeur: COUVREUR, Patrick, F-91140 Villebon Sur Yvette (FR); DESMAELE, Didier, F-94260 Fresnes (FR); RAOUANE, Mouna, F-75018 Paris (FR); MASSADE, Liliane, F-94230 Cachan (FR); MALVY, Claude, F-91800 Boussy Saint Antoine (FR)
(74) Mandataire: Le Coupanec, Pascale A.M.P.
(86) Numéro de dépôt international: PCT/FR2009/050887
(87) Numéro de publication internationale: WO 2009/150344

(56) Documents cités:
- WO-A-2006/090029
- COUVREUR PATRICK ET AL: "Squalenoyl nanomedicines as potential therapeutics" NANO LETTERS, ACS, WASHINGTON, DC, US, vol. 6, no. 11, 1 novembre 2006 (2006-11-01), pages 2544-2548, XP002489419 ISSN: 1530-6984 [extrait le 2006-10-07]
- REDDY ET AL: "A new nanomedicine of gemcitabine displays enhanced anticancer activity in sensitive and resistant leukemia types" JOURNAL OF CONTROLLED RELEASE, ELSEVIER, AMSTERDAM, NL, vol. 124, no. 1-2, 14 novembre 2007 (2007-11-14), pages 20-27, XP022343684 ISSN: 0168-3659

## Description

La présente invention concerne le domaine de la biologie moléculaire et en particulier, vise à proposer un nouveau type de véhicule utile en thérapie génique, pour la vectorisation d'acide nucléique.

La thérapie génique repose principalement sur l'utilisation d'acides nucléiques dans un but thérapeutique, contribuant ainsi à l'obtention d'« ADN-médicament ». Initialement limité au cadre des maladies génétiques héréditaires où une copie fonctionnelle du gène causal est introduite dans les cellules somatiques affectées par le déficit héréditaire, le transfert de gènes s'est étendu au traitement de maladies acquises, telles que les cancers, par exemple.

Plus récemment, la thérapie génique s'est étendue au transfert de courtes séquences d'acide nucléique dans le but, soit de modifier l'expression d'un gène (saut d'exons, par exemple), soit de réparer, par recombinaison génétique, l'anomalie génétique causale (séquences courtes d'ADN, chimères ADN/ARN, par exemple), ou encore d'inhiber l'expression d'un gène (ARNsi ou « Small Interferent RNA », oligonucléotides antisens, par exemple).

Les inventeurs de la présente invention se sont plus particulièrement intéressés à ce dernier type de stratégie thérapeutique.

L'interférence de l'ARN est un phénomène hautement conservé au cours de l'évolution. Il est basé sur un mécanisme mettant en oeuvre de petits ARN généralement double brin, appelés ARNsi, comprenant généralement de 21 à 25 nucléotides, qui, une fois transfectés dans la cellule, par complémentarité de séquences avec des ARNm (ARN messagers) bloquent leur traduction.

Ces ARNsi peuvent être transfectés en tant que tels dans la cellule ou être issus de la dégradation d'une molécule d'ARN de plus grande taille.

Dans ce dernier cas, les ARN doubles brins présents dans une cellule sont tout d'abord pris en charge par une ribonucléase de type III appelée Dicer, l'« éminceuse ». Celle-ci clive l'ARN double brin toutes les 21 à 25 paires de bases, formant ainsi des ARNsi. Dicer transfère alors les ARNsi à un complexe multiprotéique, le complexe RISC (RNA-Induced Silencing Complex). Un des brins de l'ARNsi, dit « passager », est éliminé tandis que l'autre, appelé « guide », dirige le complexe RISC vers les ARNm possédant une séquence complémentaire au brin guide. Si la complémentarité -entre l'ARNsi et l'ARNm cible est parfaite, le complexe RISC clive l'ARNm cible qui est alors dégradé et n'est donc plus traduit en protéine.

Ainsi, les ARNsi transfectés tels quels ou issus du clivage d'ARN plus long, conduisent à la mise en veille de l'expression de gènes cibles. Quelques bases non complémentaires suffisent pour empêcher le clivage. Ce mécanisme est donc très spécifique de la séquence de l'ARNsi et de sa cible, l'ARNm.

Or, de nombreux gènes sont surexprimés ou exprimés au mauvais endroit ou au mauvais moment dans de nombreuses pathologies. La possibilité de pouvoir inhiber ces expressions pathologiques est un espoir important pour soigner ces nombreuses maladies, au premier rang desquelles on trouve les cancers mais également les maladies d'origine virale ou autoimmunes.

L'utilisation des ARNsi représentent un outil intéressant pour l'étude de la fonction de certains gènes. En effet, en bloquant l'expression d'un gène cible, il est possible de déterminer, par défaut, sa fonction.

Toutefois, la mise en oeuvre *in vivo* de cette stratégie thérapeutique soulève encore beaucoup de difficultés, notamment en terme de réalisation. En effet, la transfection d'acide nucléique dans le cadre de thérapie génique se heurte principalement à trois obstacles.

Tout d'abord, pour être efficaces, les acides nucléiques doivent obligatoirement gagner les cellules cibles spécifiquement visées et franchir les membranes cellulaires, tout en n'étant pas dégradés par l'organisme. Ensuite, la transfection d'acide nucléique doit en outre être acceptable en terme de toxicité. Enfin, les molécules d'acide nucléique possèdent une stabilité *in vivo* faible.

Pour surmonter ces difficultés, plusieurs techniques de vectorisation ont déjà été proposées pour le transfert de molécules d'acides nucléiques dans la cellule.

Une première technique met en oeuvre l'adénovirus (AAV, pour *« Adeno-Associated Virus »* en anglais), qui est un virus recombinant. Un autre mode de transfection, l'électrotransfert, consiste à imposer un champ électrique au niveau des cellules considérées après y avoir injecté une molécule d'acide nucléique. En fait, bien que ces deux premières méthodes permettent d'obtenir des niveaux satisfaisants de transfert de gènes, elles présentent en revanche un inconvénient en terme de toxicité.

Une autre technique permettant précisément de s'affranchir de cet inconvénient, met à profit les vecteurs synthétiques. Des exemples de vecteurs synthétiques généralement utilisés pour la transfection d'acides nucléiques sont par exemple les lipoplexes qui sont des complexes d'acides nucléiques et de lipides cationiques, éventuellement en association avec des lipides neutres. Ces vecteurs, chargés positivement, sont essentiellement internalisés dans la cellule par le biais d'interactions électrostatiques non spécifiques. A titre d'exemple, le chitosan, un polysaccharide chargé positivement contenant des résidus β-D-glucosamine non acétylés, a été largement étudié pour son utilisation dans des systèmes de délivrance d'acide nucléique. Ainsi, il est connu de H. de Martimprey et al., Nucleic Acids Res. 2008 ; 36(1), des systèmes de délivrance d'ARNsi où l'ARNsi est adsorbé à la surface des nanoparticules de type « coeur/couronne », le coeur comprenant du poly(isobutylcyanoacrylate et la couronne du chitosan.

Toutefois, la mise en oeuvre de système de délivrance utilisant des cations lipidiques nécessitent généralement de nombreuses mises au point avant une utilisation *in vivo* pour espérer une efficacité de transfection optimale.

De plus, la formation du complexe acide nucléique/vecteur synthétique n'est pas toujours immédiate et peut être contraignante en terme de synthèse. En outre, comparés aux vecteurs viraux, les vecteurs synthétiques restent moins efficaces *in vivo* pour cibler un tissu après une injection intraveineuse. Enfin, tous ces systèmes ont, de par leur caractère cationique ? une tendance naturelle à interagir fortement avec les protéines plasmatiques chargées négativement ce qui peut entrainer des phénomènes d'agrégation et des réactions toxiques.

La présente invention a précisément pour objet de proposer un nouveau mode de vectorisation synthétique, de nature non cationique, pour la transfection d'acide nucléique, permettant de répondre aux inconvénients précités.

En particulier, la présente invention résulte de la constatation inattendue par les inventeurs que le couplage d'une molécule d'acide nucléique comportant de 10 à 40 nucléotides à un composé hydrocarboné, au moins en C₁₈, de type radical squalénoyle ou analogue de celui-ci, conduit à la formation de nanoparticules, constituant un système de délivrance efficace en termes de vectorisation et de transfection, qui plus est acceptable sur le plan de la toxicité.

WO 2006/090029 décrit déjà l'aptitude du squalène, lorsqu'il est couplé de manière covalente à la gemcitabine, une molécule hydrophile et polaire, à former spontanément des nanoparticules d'une centaine de nanomètres en milieu aqueux. Cette capacité y est notamment expliquée par le comportement amphiphile des dérivés ainsi synthétisés, la partie squalène représentant la partie hydrophobe et la partie gemcitabine la partie hydrophile.

Contre toute attente, les inventeurs ont constaté que cette aptitude du squalène et de ses analogues à former des nanoparticules pouvait également se manifester lorsque celui-ci est associé de manière covalente à une molécule d'acide nucléique de petite taille, tel qu'un ARNsi ou un oligonucléotide antisens, avec toutefois un poids moléculaire qui demeure très supérieur à celui des analogues nucléosidiques considérés dans WO 2006/090029. De façon inattendue, les nanoparticules ainsi formées s'avèrent en outre dotées d'une taille suffisamment faible (de quelques dizaines à quelques centaines de nanomètres) pour être compatibles avec une application en thérapie génique, par voie systémique.

Ainsi, la présente invention concerne selon un premier aspect, un complexe formé d'au moins une molécule d'acide nucléique comportant de 10 à 40 nucléotides, couplée de manière covalente à au moins un composé hydrocarboné au moins en C₁₈, à structure squalénique ou analogue de celui-ci.

Selon un mode de réalisation préféré de la présente invention, la molécule d'acide nucléique comprenant de 10 à 40 nucléotides est un oligonucléotide antisens ou, de façon tout particulièrement préférée, une molécule d'ARN et en particulier une molécule d'ARNsi.

Ainsi, un objet de l'invention vise un complexe conforme à l'invention dans lequel la molécule d'acide nucléique est une molécule d'ARN et en particulier une molécule d'ARNsi.

D'autres molécules d'acides nucléiques sont également susceptibles d'être véhiculées par le système de vectorisation selon l'invention, telles que les aptamères.

A ce titre, peuvent tout particulièrement être mises en oeuvre des séquences utiles pour le traitement de cancers, telles que l'ARNsi RET/PTC1 et l'ARNsi RET/PTC3 (carcinome papillaire de la thyroïde), l'ARNsi EWS-fli1 (sarcome d'Ewing), l'oligonucléotide antisens anti-ICAM et l'ARNsi anti-ICAM (Kretschmer-Kazemi Far, R. et al. Nucl. Acids Res. 2003 31:4417-4424), les ARNsi anti-Rho A et anti-Rho C (Pille et al., (2005). Anti-RhoA and anti-RhoC siRNAs inhibit the proliferation and invasiveness of MDA-MB-231 breast cancer celles in vitro and in vivo. Mol Ther 11, 267-574), les ARNsi anti-K-ras (Martin SE, et al. Multiplexing siRNAs to compress RNAi-based screen size in human cells. Nucleic Acids Res. 2007; 35(8)) et l'ARNsi inhibant l'expression de facteurs de croissance tels que le VEGF-A et le VEGF-C (pour Vascular Endothelial Growth Factor de type A ou B, en anglais), (Filleur et al., Cancer Res, 2003).

En particulier, l'ARNsi RET/PTC1 est un ARNsi ayant une séquence complémentaire de l'oncogène chimérique RET/PTC1 (pour « Papillary Thyroid Carcinoma »), résultant d'un réarrangement chromosomique et en particulier de la fusion du domaine tyrosine kinase du protooncogène RET (récepteur membranaire ayant une activité tyrosine kinase) avec l'extrémité 5' d'un autre gène, en l'occurrence H4. Cet oncogène chimérique est détecté notamment chez un sujet atteint d'un carcinome papillaire de la thyroïde.

Plusieurs ARNsi RET/PTC1 ont été criblés, sélectionnés et clonés (H. De Martimprey *et al*., 2007, *cf. infra*).

Est plus particulièrement considérée selon l'invention la séquence d'ARNsi RET-PTC 1.

Un objet de l'invention vise donc un complexe selon l'invention dans lequel la molécule d'acide nucléique est un ARNsi RET/PTC1, ayant la séquence qui suit : 5'-CGUUACCAUCGAGGAUCCAdAdA-3' (SEQ ID NO : 1).

Selon un autre objet de l'invention, les deux entités formant le complexe selon la présente invention sont couplées par une liaison covalente de type ester, éther, thioéther, disulfure, phosphate ou amide, de préférence disulfure.

Selon un mode particulier de réalisation, un complexe est formé d'au moins une molécule d'acide nucléique comprenant de 10 à 40 nucléotides, couplée de manière covalente à au moins deux composés hydrocarbonés au moins en C₁₈, à structure squalénique ou analogue de celui-ci.

Un autre objet de la présente invention vise les nanoparticules d'un complexe selon la présente invention, tel que décrit précédemment.

Selon un autre objet, la présente invention vise un procédé de préparation de nanoparticules selon la présente invention, caractérisé en ce qu'il comprend au moins :
- la dispersion d'un complexe selon l'invention dans au moins un solvant organique, par exemple un alcool comme l'éthanol, à une concentration suffisante pour obtenir, lors de l'ajout du mélange résultant, sous agitation, et généralement au goutte-à-goutte, à une phase aqueuse, la formation instantanée de nanoparticules dudit complexe en suspension dans ladite phase aqueuse, et,
- le cas échéant, l'isolement des dites nanoparticules.

De manière particulièrement préférée, l'ARNsi mis en oeuvre dans un complexe selon l'invention et dans les nanoparticules formées à partir de ce complexe est l'ARNsi RET/PTC1 (SEQ ID NO : 1).

La présente invention vise, selon un autre de ses objets, un complexe et/ou les nanoparticules correspondantes selon l'invention pour leur utilisation pour le traitement et/ou la prévention de cancers, notamment de cancers endocrines et plus particulièrement le carcinome papillaire de la thyroïde, ou encore pour le traitement et/ou la prévention de pathologies virales.

L'invention vise également des compositions pharmaceutiques comprenant au moins un complexe et/ou des nanoparticules selon l'invention, en association avec au moins un véhicule pharmaceutique acceptable.

### Composé hydrocarboné à structure squalénique

Au sens de la présente invention, un « composé à structure squalénique » est un composé comprenant au moins un radical squalénoyle notamment tel que défini ci-après.

Au sens de la présente invention, un « radical squalénoyle » est apte à reproduire les propriétés d'auto-organisation manifestées par le squalène et/ou ses dérivés lorsqu'il est mis en présence d'un milieu aqueux.

En particulier un « radical squalénoyle » selon l'invention peut être schématisé par la structure hydrocarbonée, linéaire, formée d'unités isoprène, pouvant être figurée par la formule (I) qui suit : 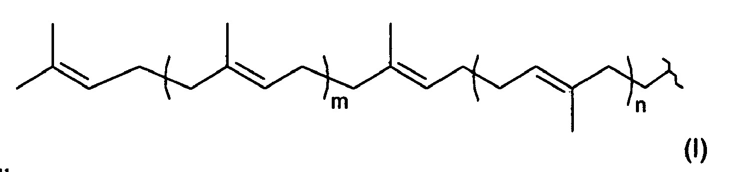 dans laquelle :
- m = 1, 2, 3, 4 ou 5 ;
- n = 0, 1, 2, 3, 4 ou 5 ; et
- 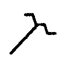 représente la liaison vers le reste du complexe avec l'acide nucléique.

Selon un mode de réalisation préféré de la présente invention, on met en oeuvre un radical squalénoyle de formule (I') qui suit, correspondant à un radical de formule (I) précédent dans lequel n = 0 :

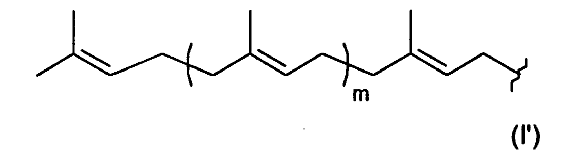

Plus particulièrement un radical squalénoyle de formule (I) selon l'invention pour lequel m = 1 et n = 3 peut présenter la structure du squalène ou de ses dérivés, de formule suivante :

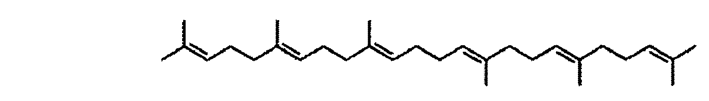

Selon un mode de réalisation préféré de l'invention, on met en oeuvre un radical de formule (I) dans laquelle m = 1 et n = 2.

Ainsi, un objet de l'invention concerne un complexe conforme à la présente invention dans lequel le composé hydrocarboné au moins en C₁₈, à structure squalénique ou analogue est figuré par un radical de formule (I) tel que défini précédemment.

A titre illustratif de ces composés hydrocarbonés, on peut plus particulièrement citer l'acide squalénique et ses dérivés tel que l'acide 1, 1', 2-tris-norsqualénique. On peut également citer, à titre d'exemple, le squalène (encore appelé spiracène ou sirprène), isoprénoïde à 30 atomes de carbone et 50 atomes d'hydrogène (nom chimique : (E) 2, 6, 10, 15, 19, 23-Hexaméthyl-2, 6, 10, 14, 18, 22-tétracosahexène). Le squalène est un intermédiaire essentiel de la biosynthèse du cholestérol.

Un objet de l'invention vise également des complexes conformes à la présente invention contenant au moins l'un des composés hydrocarbonés particuliers précités.

En particulier, un objet de la présente invention vise un complexe selon l'invention dans lequel le composé hydrocarboné au moins en C₁₈, à structure squalénique est l'acide 1, 1'1 2-tris-norsqualénique.

Comme les inventeurs l'ont constaté, ce radical squalénoyle est particulièrement important dans le cadre de la présente invention car il manifeste spontanément, lorsqu'il est mis en présence d'un milieu polaire et plus particulièrement l'eau, une conformation compactée.

De manière inattendue, les inventeurs ont constaté que cette aptitude demeure lorsqu'un tel radical est associé et notamment lié de manière covalente à une molécule d'acide nucléique comprenant de 10 à 40 nucléotides. Il s'en suit la génération d'une architecture compactée à l'état de nanoparticules dans laquelle les deux entités acide nucléique/composé à structure squalénique sont intimement imbriquées l'une dans l'autre.

Au sens de la présente invention, « analogue » désigne un composé hydrocarboné d'une part, apte à reproduire le comportement d'un composé à structure squalénique lorsqu'il est mis en présence d'un milieu polaire et d'autre part, capable de reproduire cette aptitude lorsqu'il est lié à une molécule d'acide nucléique selon l'invention. Sont notamment couverts sous cette définition les formes substituées des dérivés du squalène et en particulier l'acide squalénique et ses dérivés, notamment de substitution. A titre d'exemple de dérivé de l'acide squalénique, on peut citer l'acide 1,1',2-tris-norsqualénique.

Généralement au moins un composé hydrocarboné à structure squalénique est liée de manière covalente à une molécule d'acide nucléique. Bien entendu, le nombre de molécules de dérivé hydrocarboné susceptible d'interagir avec une molécule d'acide nucléique peut être supérieur à 1.

### Complexe acide nucléique/composé à structure squalénique

La formation du complexe acide nucléique/composé à structure squalénique, nécessite que les deux entités du complexe portent des fonctions susceptibles de former une liaison covalent ou un bras de liaison, tels que décrits précédemment.

Le composé hydrocarboné à structure squalénique est généralement porteur d'une fonction susceptible de réagir avec une fonction présente sur la molécule de l'acide nucléique considérée de manière à établir un lien covalent entre les deux entités par exemple de type ester, éther, thioéther, disulfure, phosphate ou amide, formant ainsi un complexe covalent. Avantageusement, il s'agit d'une fonction thiol. Auquel cas, le composé hydrocarboné à structure squalénique est l'acide 1,1',2-tris-norsqualénique ou l'un de ses dérivés et en particulier amides ou esters.

Selon une variante de réalisation, le lien covalent existant entre les deux types de molécules peut être figuré par un espaceur ou encore bras de liaison. Un tel bras peut notamment s'avérer utile pour augmenter la force de l'interaction acide nucléique/radical squalénoyle.

Un tel bras permet précisément d'introduire *via* chacune des deux extrémités de son squelette les fonctions adéquates, c'est-à-dire possédant respectivement l'affinité réactionnelle attendue, l'une pour la fonction présente sur le dérivé à structure squalénique et l'autre pour la fonction présente sur la molécule d'acide nucléique considérée.

On peut également envisager que ce bras de liaison possède en outre au niveau de son squelette une fonction labile, propice ultérieurement à la séparation du composé à structure squalénique de la molécule d'acide nucléique considérée. Il peut par exemple s'agir d'un motif peptidique reconnaissable par une enzyme.

Les motifs de type bras de liaison sont bien connus de l'homme de l'art et leur mise en oeuvre relève clairement de ses compétences.

A titre représentatif des bras de liaison envisageables selon l'invention, on peut notamment citer les motifs (poly)aminoacides, polyols, saccharidiques, et polyéthylèneglycol (polyétheroxides) de faible poids moléculaire.

Ainsi, au sens de la présente invention, un « lien covalent » figure de préférence une liaison covalente notamment telle que précisée ci-dessus, mais couvre également un lien covalent figuré par un bras de liaison tel que défini précédemment.

Ainsi, le complexe covalent selon la présente invention peut être figuré par le composé de formule (II) qui suit : 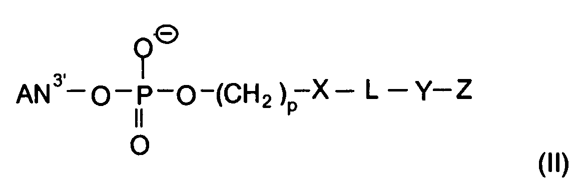
- AN représente une molécule d'acide nucléique comprenant de 10 à 40 nucléotides, dont l'extrémité 3' est reliée au reste du complexe,
- X représente un lien covalent entre les deux entités, et plus particulièrement un lien de type ester, éther, thioéther, disulfure, phosphate ou amide ;
- L représente un bras de liaison, choisi de préférence parmi les motifs chaîne alkyle saturée, ou encore (poly)aminoacides, polyols, saccharidiques, et polyéthylèneglycol (polyétheroxides) de faible poids moléculaire, à savoir un poids moléculaire variant de 40 à 500 daltons.
- Y représente un lien covalent, et plus particulièrement un lien de type ester, amide, thioéther ou phosphate ;
- p représente 0, 1, 2, 3 ou 4,
- Z représente radical squalénoyle ou dérivé de celui-ci, de formule (I) ou (I') précédente.

Au sens de la présente invention :
- on entend par « chaîne alkyle saturée », un radical alkyl saturé linéaire ou ramifié pouvant comporter de 1 à 8 atomes de carbone dans lequel deux liaisons à un atome d'hydrogène sont remplacées par deux liaisons covalentes au reste de la molécule,
- on entend par « motif saccharidique », un radical comprenant au moins un radical choisi parmi les trioses (glycéraldéhyde, dihydroxyacétone), tétroses (érythrose, thréose, érythrulose,), pentoses (arabinose, lyxose, ribose, désoxyribose, xylose, ribulose, xylulose), hexoses (allose, altrose, galactose, glucose, gulose, idose, mannose, talose, fructose, psicose, sorbose, tagatose, heptoses (mannoheptulose, sedoheptulose), octose (octolose, 2-céto-3-désoxy-manno-octonate, isonoses (sialose), et
- on entend par « motif (poly)aminoacide », un motif ayant au moins une unité : 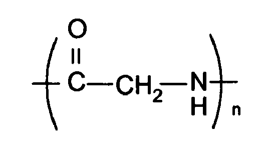
dans laquelle n est supérieur ou égal à 1.

La présente invention concerne plus particulièrement un complexe, figuré par le composé de formule (IIA) qui suit : 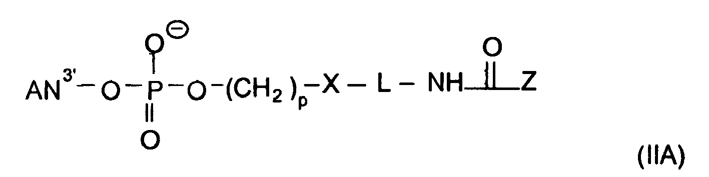 dans laquelle :
- AN, X, L, Z et p ont les mêmes définitions que pour le composé de formule (II).

Dans les exemples de formule (II) et (IIA), L représente de préférence une chaîne alkyle saturée telle que définie précédemment.

Dans les complexes de formules (II) et (IIA), AN est de préférence une molécule d'acide ribonucléique, comprenant de préférence de 19 à 25 nucléotides.

De façon particulièrement préférée, l'acide ribonucléique est un ARNsi, notamment un ARNsi RET/PTC1 de séquence correspondant à SEQ ID NO : 1.

En particulier, un objet de la présente invention vise un complexe de formule (II) dans lequel X est un lien covalent disulfure et Z représente un radical de formule (I) dans lequel m représente 1 et n représente 2.

La réaction nécessaire à l'établissement d'au moins une liaison covalente entre au moins une molécule d'acide nucléique considéré et au moins un composé à structure squalénique ou analogue peut être effectuée selon des conditions standards et sa réalisation relève donc clairement des connaissances de l'homme de l'art.

Cette réaction est généralement réalisée en solution en présence et en excès d'au moins un composé à structure squalénique par rapport à la molécule d'acide nucléique considérée, par exemple à raison de deux équivalents, selon les conditions standards requises pour faire interagir les deux fonctions spécifiques portées par chacune des deux entités.

Préalablement à cette réaction, chacune des deux entités, la molécule d'acide nucléique d'une part, et le composé hydrocarboné d'autre part sont modifiées afin de porter une fonction susceptible d'établir un lien covalent entre elles. De préférence, chacune des deux molécules est porteuse d'une fonction thiol afin d'établir un pont disulfure entre elles.

La fonctionnalisation d'une molécule d'ARN est largement documentée dans l'état de la technique. Par exemple, elle peut être fonctionnalisée par une fonction thiol, par analogie avec la méthode décrite dans Gnaccarini et al., J. Am. Chem. Soc. 2006, 128, 8063-8067, c'est-à-dire à l'aide d'un phosphoramidite approprié. Dans le cas présent, ce type de fonctionnalisation est greffé à l'extrémité 3' de la molécule d'ARNsi.

Pour sa part, le composé à structure squalénique est fonctionnalisé, notamment par une fonction thiol, par analogie avec la méthode décrite dans Elbright et al., Biochemistry 1992, 31, 10664-10670, c'est-à-dire en utilisant un dérivé pyridyl disulfure.

La conjugaison des deux partenaires ainsi fonctionnalisés est réalisée par analogie avec la méthode de couplage décrite dans Sengle et al. Biorg. & Med. Chem., 2000, 8, 1317-1329, encore appelée « *GMPS-coupling method* ».

Un objet de la présente invention vise donc également un complexe conforme à l'invention, figuré par les formules (II) ou (IIA) telles que définies précédemment.

### Nanoparticules selon l'invention

Comme précisé précédemment, le couplage covalent d'une molécule d'au moins une molécule d'acide nucléique considérée selon l'invention avec au moins une molécule d'un composé hydrocarboné à structure squalénique est de nature à conférer à l'acide nucléique ainsi complexé une aptitude à s'organiser sous une forme compactée dans un milieu solvant polaire, conduisant ainsi à la formation de nanoparticules.

D'une manière générale, les nanoparticules ainsi obtenues possèdent une taille moyenne variant de 30 à 500 nm, et en particulier de 50 à 250 nm, voire de 100 à 400 nm mesurée par diffusion de la lumière à l'aide du nanosizer Coulter^{®} N4MD, Coulter Electronics, Hialeah, USA.

Un objet de l'invention vise des nanoparticules conformes à l'invention dont la taille moyenne varie de 30 à 500 nm, en particulier de 50 à 250 nm, voire de 100 à 400 nm.

Ainsi, l'interaction d'une molécule d'acide nucléique, très hydrosoluble considérée selon l'invention avec un dérivé squalénoyle ou l'un de ses analogues comme par exemple, l'acide 1,1',2-tris-norsqualénique, confère à ladite molécule d'acide nucléique des caractéristiques physico-chimiques suffisantes pour lui conférer une aptitude à former des particules dont la taille s'avère compatible pour une administration parentérale et notamment par voie intraveineuse.

Comme indiqué précédemment, les nanoparticules selon la présente invention peuvent être obtenues par une étape de dispersion d'un complexe selon l'invention, c'est-à-dire formé préliminairement par couplage d'au moins un composé à structure squalénique ou analogue à au moins une molécule d'acide nucléique, selon l'invention, dans au moins un solvant organique, par exemple un alcool comme l'éthanol, à une concentration suffisante pour obtenir, lors de l'ajout du mélange résultant, sous agitation, et généralement au goutte-à-goutte, à une phase aqueuse. La formation instantanée de nanoparticules du dit dérivé en suspension dans la dite phase aqueuse, suivi, le cas échéant, de l'isolement des dites nanoparticules.

La réaction peut généralement être réalisée à température ambiante. Quelle qu'elle soit, la température de réaction ne doit pas affecter l'activité de la molécule d'acide nucléique considérée. Le procédé de préparation des nanoparticules selon l'invention est particulièrement avantageux dans la mesure où il ne requiert pas obligatoirement la présence de tensioactifs.

Selon un mode de réalisation particulier, les nanoparticules sont obtenues sous forme d'une suspension aqueuse.

Cette propriété est particulièrement appréciable dans la mesure où un grand nombre de tensioactifs ne s'avèrent pas compatibles avec une application *in vivo.*

Toutefois, il est entendu que l'usage de tensioactifs, généralement avantageusement dénués de toute toxicité, est envisageable dans le cadre de l'invention. Ce type de tensioactifs peut par ailleurs permettre d'accéder à des tailles encore plus réduites lors de la formation de nanoparticules. A titre illustratif et non limitatif de ce type de tensioactifs susceptibles d'être utilisés dans la présente invention, on peut notamment citer des copolymères de polyoxyéthylène-polyoxypropylène, des dérivés phospholipidiques et des dérivés lipophiles du polyéthylène glycol.

Comme dérivé lipophile du polyéthylène glycol, on peut mentionner par exemple le polyéthylène glycol cholestérol. Comme exemple des copolymères bloc polyoxyéthylène-polyoxypropylène, on peut particulièrement citer les copolymères triblocs polyoxyéthylène- polyoxypropylène- polyoxyéthylène, encore appelés poloxamères^{®}, pluronics^{®} ou synperonics et qui sont commercialisés, notamment, par la société BASF.

Apparentés à ces familles de copolymères, les poloxamines, qui sont constitués de segments hydrophobes (à base de polyoxypropylène), de segments hydrophiles (à base de polyoxyéthylène) et d'une partie centrale dérivant du motif éthylène diamine peuvent également être employés.

Les nanoparticules selon l'invention sont bien entendu susceptibles de porter en surface une multitude de fonctions réactives, à l'image des fonctions hydroxyle ou amine par exemple. Il est donc envisageable de fixer à ces fonctions toutes sortes de molécules, notamment par des liaisons covalentes.

A titre illustratif et non limitatif de ce type de molécules susceptibles d'être associées aux nanoparticules, on peut notamment citer les molécules de type marqueur, les composés susceptibles d'assurer une fonction de ciblage, ainsi que tout composé apte à leur conférer des caractéristiques pharmacocinétiques particulières. En ce qui concerne ce dernier aspect, on peut ainsi envisager de fixer en surface de ces nanoparticules des dérivés lipophiles du polyéthylène glycol, comme par exemple le conjugué polyéthylène glycol/cholestérol, le polyéthylène glycol-phosphatidyléthanolamine ou mieux encore le polyéthylène glycol/squalène. En effet, compte-tenu de l'affinité naturelle des résidus de squalène entre eux, le conjugué polyéthylène glycol/squalène s'associe, en l'espèce, avec les nanoparticules selon l'invention, et conduit ainsi à la formation de nanoparticules revêtues en surface de polyéthylène glycol. Par ailleurs, et comme mentionné précédemment, le conjugué polyéthylène glycol/squalène agit avantageusement lors du processus de formation des nanoparticules selon l'invention, comme tensioactif du fait de son comportement amphiphile et stabilise donc la solution colloïdale, réduisant ainsi la taille des nanoparticules formées. Un enrobage de surface à base de tels composés et en particulier le polyéthylène glycol ou le conjugué polyéthylène glycol /cholestérol ou le conjugué polyethylèneglycol/squalène, est en effet avantageux pour conférer une rémanence vasculaire accrue en raison d'une réduction significative de la capture des nanoparticules par les macrophages hépatiques.

Selon un mode de réalisation avantageux, les nanoparticules selon l'invention sont formulées à l'état de dispersion aqueuse en vue de leur administration généralement par voie systémique.

Selon un autre mode de réalisation avantageux, cette dispersion aqueuse contient moins de 5 % en poids, voire moins de 2 % en poids et plus particulièrement est dénuée de tensioactif ou analogue tels que par exemple les polyéthylène glycols, le polyglycérol et leurs dérivés, tels les esters par exemple.

Selon un autre mode de réalisation avantageux, cette dispersion aqueuse contient moins de 5 % en poids, voire moins de 2 % en poids en alcool en C₂ à C₄ tel que par exemple l'éthanol.

Selon un autre mode de réalisation avantageux, cette dispersion aqueuse possède intrinsèquement une viscosité compatible avec une administration par voie intraveineuse.

Ainsi, la formulation en milieu aqueux de l'acide nucléique considéré à l'aide de l'acide squalénique à l'état de nanoparticules hydrodispersibles permet, avantageusement, d'obtenir une suspension de nanoparticules sans autre additif que le dextrose 5 % nécessaire pour obtenir l'isotonie de la suspension injectable.

Comme indiqué précédemment, la présente invention vise également l'utilisation d'au moins une nanoparticule selon l'invention dans des compositions pharmaceutiques.

Un autre aspect de l'invention concerne donc une composition pharmaceutique comprenant au moins, au titre de matière active, un complexe conforme à la présente invention notamment sous la forme de nanoparticules. Les complexes conformes à la présente invention peuvent y être associés avec au moins un véhicule pharmaceutiquement acceptable.

A titre d'exemples de formulations pharmaceutiques compatibles avec les compositions selon l'invention, on peut notamment citer :
- les injections ou perfusions intraveineuses ;
- les solutions salines ou d'eau purifiée ;
- les compositions pour inhalation ;
- les capsules, dragées, cachets et sirops incorporant notamment à titre de véhicule, de l'eau, du phosphate de calcium, des sucres, tels que lactose, dactrose ou mannitol, du talc, de l'acide stéarique, de l'amidon, du bicarbonate de sodium et/ou de la gélatine.

Lorsque les complexes et/ou nanoparticules sont utilisés en dispersion dans une solution aqueuse, ils peuvent être associés à des excipients de type agent séquestrant ou chélatant, antioxydant, agents modifiant le pH et/ou agents tampons.

Outre les composés précités, les compositions pharmaceutiques selon l'invention peuvent contenir des agents de type conservateurs, des agents mouillants, des agents solubilisants et des agents de coloration.

De telles compositions selon l'invention au regard de la nature de l'acide nucléique considéré à l'état de nanoparticules selon l'invention, peuvent s'avérer particulièrement utiles pour le traitement et/ou la prévention de cancers chez les mammifères. On peut citer par exemple, sans toutefois y être limité, les carcinomes, sarcomes, cancers hématopoïétiques, le cancer du sein, du côlon, du rectum, du pancréas, des testicules, de l'utérus, du tractus gastro-intestinal, des poumons, des ovaires, de la prostate de la bouche, du cerveau, de la tête, du coup, de la gorge, des reins, des os, du foie, de la rate, de la vessie, de la peau, du larynx, des voies nasales, des cancers lié au SIDA, les cancers endocrines, le myélome multiple (ou cancer de la moelle osseuse), les leucémies, le sarcome de Kaposi, les lymphomes, tels que la maladie de Hodgkin ou le lymphome non-hodgkinien.

Par « traitement », on entend au sens de l'invention, une inhibition ou une réduction du taux de prolifération de cellules cancéreuses, permettant une rémission partielle ou totale.

Ainsi, dans le cas où l'acide nucléique est l'ARNsi RET/PTC1 (SEQ ID NO :1), de telles compositions sont utiles pour le traitement et/ou la prévention de cancers endocrines tels que les cancers de la thyroïde et plus particulièrement le carcinome papillaire de la thyroïde.

Ainsi, les complexes et/ou nanoparticules selon la présente invention sont utiles pour la préparation d'une composition pharmaceutique destinée au traitement et/ou à la prévention de cancers, tels que les carcinomes, sarcomes, cancers hématopoïétiques, le cancer du sein, du côlon, du rectum, du pancréas, des testicules, de l'utérus, du tractus gastro-intestinal, des poumons, des ovaires, de la prostate de la bouche, du cerveau, de la tête, du coup, de la gorge, des reins, des os, du foie, de la rate, de la vessie, de la peau, du larynx, des voies nasales, des cancers lié au SIDA, les cancers endocrines, le myélome multiple (ou cancer de la moelle osseuse), les leucémies, le sarcome de Kaposi, les lymphomes, tels que la maladie de Hodgkin ou le lymphome non-hodgkinien.

En particulier, les complexes et/ou nanoparticules selon la présente invention sont utiles pour la préparation d'une composition pharmaceutique destinée au traitement et/ou à la prévention de cancers, tels que les cancers endocrines et plus particulièrement le carcinome papillaire de la thyroïde.

Les complexes ou nanoparticules conformes à la présente invention peuvent être administrés par toutes les voies conventionnelles. Toutefois, comme précisé précédemment, compte tenu de la faible taille de leurs particules, ils sont administrables sous la forme d'une suspension aqueuse par voie intraveineuse et donc compatibles avec la microcirculation vasculaire.

Pour des raisons évidentes, les quantités en dérivés selon l'invention susceptibles d'être mis en oeuvre sont susceptibles de varier significativement selon le mode d'utilisation et la voie retenue pour leur administration.

En revanche, pour une administration topique et tout particulièrement pour le traitement des mélanomes, on peut envisager de formuler au moins un complexe et/ou nanoparticule conforme(s) à la présente invention à raison de 1 à 20 % en poids, voire plus, par rapport au poids total de la formulation pharmaceutique considérée.

Il est également possible de co-administrer au moins un complexe et/ou nanoparticule conforme(s) à la présente invention avec au moins une autre matière active susceptible d'être également bénéfique à l'égard de la pathologie considérée.

A titre représentatif de ces matières actives susceptibles d'être combinées aux complexe et/ou nanoparticule conforme(s) à la présente invention, on peut notamment citer d'autres molécules ou macromolécules anticancéreuses ou cytostatiques (par exemple sels de platine, antracyclines, poisons du fuseau mitotique, inhibiteurs de topoisomérases, de kinases ou de métalloprotéases), des agents anti-inflammatoires de type corticoïde (par exemple dexaméthasone) ou non corticoïde ou encore des molécules à activité immuno-adjuvante (par exemple anticorps à activité anticancéreuse). En particulier, à titre d'association privilégiée, on peut citer l'association d'au moins un complexe et/ou au moins une nanoparticule selon la présente invention avec au moins un composé choisi parmi cisplatine, carboplatine, tamoxifène, épirubicine, leuprolide, bicalutamide, implant de goserelin, irinotécan, gemcitabine, sargramostime ou leurs sels pharmaceutiquement acceptable. Ces sels peuvent être préparés avec des acides pharmaceutiquement acceptables, c'est-à-dire, compatibles notamment en terme de toxicité, pour une utilisation pharmaceutique.

L'association avec l'hyperthermie utilisée dans certaines chimiothérapies peut être envisagée.

Les complexes et/ou nanoparticules conformes à la présente invention peuvent également être combinés aux thérapies chirurgicales et/ou aux radiations pour le traitement du cancer.

Les exemples qui suivent illustrent la présente invention sans pour autant y être limitée.

Les spectres infrarouges sont obtenus par mesure sur un solide ou un liquide pur en utilisant un spectromètre de Fourier (Transform Bruker Vector^{®} 22). Seules les absorptions significatives sont relevées.

Les rotations optiques ont été mesurées à l'aide d'un polarimètre Perkin-Elmer^{®} 241, à une longueur d'onde de 589 nm.

Les spectres RMN ¹H et ¹³C ont été enregistrés en utilisant un spectromètre Bruker AC^{®} 200P (à 200 MHz et 50 MHz, respectivement pour ¹H et ¹³C) ou Bruker Avance^{®} 300 (à 300 MHz et 75 MHz, respectivement pour ¹H et ¹³C).

Les spectres de masse ont été enregistrés en utilisant un appareil Bruker Esquire-LC^{®}.

L'analyse de chromatographie sur couche mince a été réalisée sur des plaques recouvertes au préalable par du gel de silice 60F₂₅₄ (couche de 0,25 mm).

La colonne de chromatographie a été réalisée sur gel de silice 60 (Merck, 230-400 mesh ASTM).

Toutes les réactions mettant en oeuvre des composés sensibles à l'air ou à l'eau ont été conduites sous hotte.

### Exemple 1 :

### a) Fonctionnalisation du squalène (obtention du N-[2-(pyridin-2-yldithio)ethyl] 1,1',2-tris-norsqualénamide)

La fonctionnalisation du squalène peut être schématisée selon le schéma 1 ci-après.

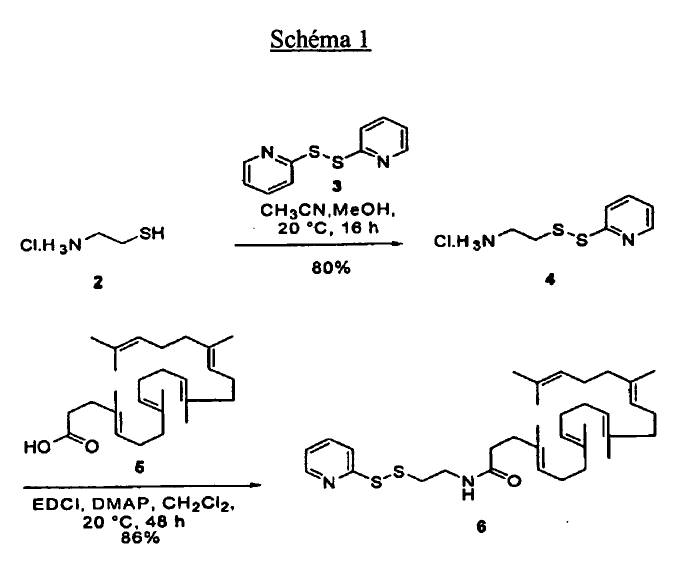

Comme indiqué dans le schéma **1** ci-dessus, pour effectuer la fonctionnalisation du squalène, la thioéthanolamine **2** est activée par le 2,2'-dithiobispyridine **3**, selon les méthodes bien connues de l'homme du métier et en particulier, par analogie avec le protocole décrit dans Elbright et al., Biochemistry 1992, 31, 10664-10670, pour obtenir le chlorhydrate de 2-(pyridin-2-yldithio)éthanamine **4**.

Le dérivé **4** est ensuite condensé avec l'acide 1,1',2-tris-norsqualènique **5** par action du chlorhydrate de 1-éthyl-3-(3-diméthylaminopropyl)carbodimide (EDCI) pour donner le N-[2-(pyridin-2-yldithio)ethyl] 1,1',2-tris-norsqualénamide **6**.

En particulier, à une solution d'acide 1,1',2-tris-norsqualènique **5** (400 mg, 1 mmol) dans le dichlorométhane anhydre (5 mL) et sous atmosphère inerte, on ajoute 226 mg (1 mmol) de 2-(pyridin-2-yldithio)ethanamine **4**, 200 mg de triethylamine (2 mmol) et 286 mg d'EDCI (1.5 mmol). Le mélange est agité à température ambiante durant 48 h puis repris dans 10 mL d'eau et extrait au dichlorométhane (3 X 20 mL). Les phases organiques sont rassemblées, lavées par une solution aqueuse saturée en NaCl, séchées sur MgSO₄ et concentrées sous pression réduite. Le résidu est purifié par chromatographie sur gel de silice en éluant par un mélange cyclohexane acétate d'éthyle 3:1.

On recueille 490 mg d'amide **6** sous forme d'une huile incolore;
IR (film) ν = 3288, 2912, 2851, 1648 (CONH), 1637 (C=C, weak), 1575, 1543, 1446 cm⁻¹;
¹H NMR (CDCl₃, 300 MHz) δ = 8,49 (d, *J* = 4,8 Hz, 1 H), 7,62 (td, *J* = 7,6, 1,8 Hz, 1 H), 7,52 (dd, *J* = 8.1, 0.9 Hz, 1 H), 7,13 (ddd, *J* = 5,0, 6,0, 0,9 Hz, 1 H), 7,03 (m, 1 H, NHCO), 5,18-5,09 (m, 5 H), 3,53 (q, *J* = 5,6 Hz, 2 H, C*H*₂NCO), 2,90 (t, *J* = 5,6 Hz, 2 H, C*H*₂SS), 2,15-1,95 (m, 20 H), 1,67 (s, 3 H), 1,63 (s, 3 H), 1,59 (s, 12 H);
¹³C NMR (50 MHz, CDCl₃) δ = 172,8 (CO), 159,2 (N=C-S), 149,2 (N=CH), 136,9 (CH), 135,0 (C), 134,8 (2 C), 133,5 (C), 131,2 (C), 125,2 (CH), 124,3 (2 CH), 124,2 (2 CH), 1212 (CH), 120,9 (CH), 39,7 (CH₂), 39,6 (2 CH₂), 38,8 (CH₂), 37,1 (CH₂), 35,4 (CH₂), 35,3 (CH₂), 28,2 (2 CH₂), 26,7 (CH₂), 26,6 (CH₂), 25,6 (CH₃), 17,6 (CH₃), 16,0 (2 CH₃), 15,8 (2 CH₃);
MS (+ESI, MeOH): *m*/*z* (%): 569 (69) [*M* + H]⁺, 591 (100) [*M* + Na]⁺;

### b) Fonctionnalisation de l'ARNsi RET/PTC1 (SEQ ID NO : 1)

L'ARNsi RET/PTC1 (SEQ ID NO : 1) est obtenu par synthèse de la séquence d'oligonucléotides, commandés à la société EUROGENTEC (Seraing, Belgique).

Une telle synthèse est effectuée selon les méthodes classiques, bien connues de l'homme du métier.

L'ARNsi RET/PTC1 est fonctionnalisé par une fonction thiol, en particulier figurée par le radical 3-mercapto-propyl, selon les méthodes bien connues de l'homme du métier, tel qu'indiqué précédemment.

### Exemple 2 : Conjugaison de l'ARNsi RET/PTC1 (SEQ ID NO : 1) avec le N-[2-(pyridin-2-yldithio)ethyl] 1,1',2-tris-norsqualénamide.

La conjugaison de l'ARNsi RET/PTC1 avec le N-[2-(pyridin-2-yldithio)ethyl] 1,1',2-tris-norsqualénamide peut être illustrée par le schéma 2 qui suit :

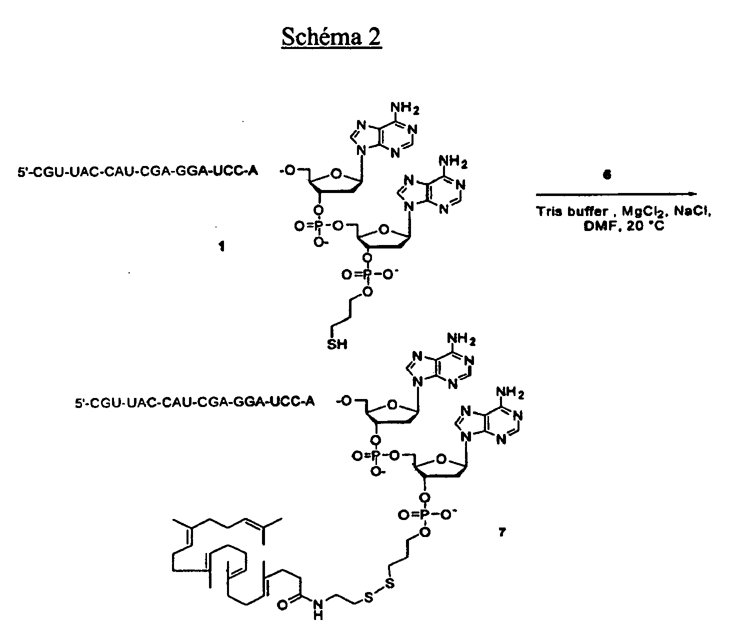

Comme indiqué dans le schéma 2 ci-dessus, la conjugaison de l'ARNsi RET/PTC1 (SEQ ID NO :1) **1** avec le N-[2-(pyridin-2-yldithio)ethyl] 1,1',2-tris-norsqualénamide 6 est réalisée par incubation à température ambiante, dans le diméthylformamide (DMF) aqueux, en milieu tamponné. L'excès du composé **6** est ensuite éliminé et le résidu obtenu est lavé avec un solvant organique, puis purifié par HPLC.

En particulier, à une solution 0.15 mM d'ARNsi **1** dans l'eau MilliQ (333 µL, 0.34 mg, 0.050 µM), on ajoute séquentiellement 633 µL d'une solution tampon 100 mM en acétate d'ammonium et 33 µL d'une solution 150 mM du dérivé pirydyldisulfure **6** tel que préparé à l'exemple 1a (2.8 mg, 5 µM) dans le DMF fraîchement distillé. Le mélange est incubé durant 10 min à 20 °C puis concentré sous pression réduite (0.5 Torr, 20 °C). Le résidu 7, correspondant au complexe brut ARNsi RET/PTC1/amido 1, 1',2-tris-norsqualène, est lavé par de l'éther éthylique (3 X 2 mL) et concentré sous vide.

Le résidu 7 est repris dans l'eau et lyophilisé pour donner 340 µg d'un solide blanc.

MS (-ESI, CH₃CN, Et₃N): *mlz* (%): 7254 (55) [*M* - H]⁻

### Exemple 3: Préparation des nanoparticules (Purification dans l'eau du complexe obtenu selon l'exemple 2)

On ajoute 500 µl d'eau milliQ^{®} au lyophilisat d'ARNsi conjugué au squalène (ARNsi - SQ), puis on centrifuge à 12000 rpm (4 °C 10 min).

Le culot de centrifugation est ensuite dissout dans 500 µl d'éthanol. La solution éthanolique est ensuite ajoutée goutte à goutte à de l'eau MilliQ^{®} contenant 5 % de dextrose sous agitation magnétique qui est maintenue 5 min. La suspension nanoparticulaire ainsi formée est ensuite transférée dans un ballon pour évaporer l'éthanol par Rotavapor. On ajuste ensuite avec de l'eau jusqu'au volume 1 ml (sur la balance).

La taille des nanoparticules est mesurée par diffusion de la lumière à l'aide du Nanosizer et trouvée égale à 355 nm.

## Revendications

1. Complexe formé d'au moins une molécule d'acide nucléique comportant de 10 à 40 nucléotides, couplée par une liaison covalente ou un bras de liaison à au moins un composé hydrocarboné au moins en C₁₈, à structure squalénique ou un analogue de celui-ci de structure hydrocarbonée, linéaire, formée d'unités isoprènes.

2. Complexe selon la revendication précédente dans lequel le composé hydrocarboné au moins en C₁₈, à structure squalénique ou analogue de celui-ci est figuré par le radical de formule (I) qui suit : 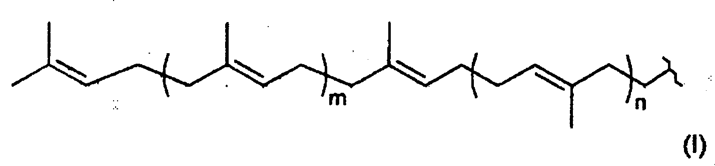 dans laquelle :
- m = 1, 2, 3, 4 ou 5 ;
- n = 0, 1, 2, 3, 4 ou 5 ; et
- 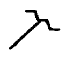 représente la liaison vers le reste du complexe avec l'acide nucléique.

3. Complexe selon l'une quelconque des revendications précédentes dans lequel le composé hydrocarboné au moins en C₁₈, à structure squalénique est l'acide 1,1',2-tris-norsqualénique.

4. Complexe selon l'une quelconque des revendications précédentes dans lequel la molécule d'acide nucléique comprenant de 10 à 40 nucléotides est une molécule d'ARN.

5. Complexe selon l'une quelconque des revendications précédentes dans lequel la molécule d'ARN est une molécule d'ARNsi.

6. Complexe selon la revendication précédente, dans lequel l'ARNsi est l'ARNsi RET/PTC1.

7. Complexe selon la revendication précédente, **caractérisé en ce que** l'ARNsi RET/PTC1 a une séquence nucléotidique qui suit :
5'-CGUUACCAUCGAGGAUCCAdAdA-3' (SEQ ID NO : 1).

8. Complexe selon l'une quelconque des revendications précédentes, dans lequel le couplage covalent est une liaison covalente de type ester, éther, thioéther, disulfure, phosphate ou amide, de préférence disulfure.

9. Complexe selon l'une quelconque des revendications précédentes, figuré par la formule générale (II) qui suit : 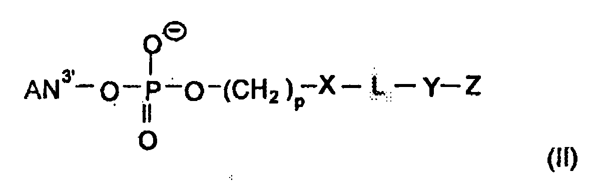
- AN représente une molécule d'acide nucléique comprenant de 10 à 40 nucléotides, dont l'extrémité 3' est reliée au reste du complexe,
- X représente un lien covalent entre les deux entités, et plus particulièrement un lien de type ester, éther, thioéther, disulfure, phosphate ou amide ;
- L représente un bras de liaison, choisi de préférence parmi les motifs chaîne alkyle saturée, (poly)aminoacides, polyols, saccharidiques, et polyéthylèneglycol (polyétheroxides) de faible poids moléculaire.
- Y représente un lien covalent, et plus particulièrement un lien de type ester, amide, thioéther ou phosphate ;
- p représente 0, 1, 2, 3 ou 4,
- Z représente un radical squalénoyle ou dérivé de celui-ci, de formule (I) tel que défini à la revendication 2.

10. Complexe selon la revendication précédente, dans lequel X est un lien covalent disulfure et Z représente un radical de formule (I) dans lequel m représente 1 et n représente 2.

11. Nanoparticules d'un complexe tel que décrit dans l'une quelconque des revendications 1 à 10.

12. Nanoparticules selon la revendication précédente, dont la taille moyenne varie de 30 à 500 nm, en particulier de 50 à 250 nm, voire de 100 à 400 nm.

13. Procédé de préparation de nanoparticules selon l'une quelconque des revendications 11 ou 12, **caractérisé en ce qu'**il comprend au moins :
- la dispersion d'un complexe selon l'une quelconque des revendications 1 à 10, dans au moins un solvant organique, à une concentration suffisante pour obtenir, lors de l'ajout du mélange résultant, sous agitation, à une phase aqueuse, la formation instantanée de nanoparticules dudit complexe en suspension dans ladite phase aqueuse, et,
- le cas échéant, l'isolement desdites nanoparticules.

14. Nanoparticules selon l'une des revendications 11 ou 12, pour leur utilisation pour le traitement et/ou la prévention de cancers, notamment de cancers endocrines et plus particulièrement le carcinome papillaire de la thyroïde.

15. Composition pharmaceutique comprenant au moins un complexe tel que décrit dans l'une quelconque des revendications 1 à 10 et/ou des nanoparticules telles que décrites dans l'une des revendications 11 ou 12, en association avec au moins un véhicule pharmaceutique acceptable.

## Claims

1. A complex formed by at least one molecule of nucleic acid comprising from 10 to 40 nucleotides, covalently coupled or coupled with a linker arm to at least one hydrocarbon-based compound, that is at least a C₁₈ hydrocarbon-based compound, having a squalene structure or an analog thereof having a linear hydrocarbon-based structure formed by isoprene units.

2. The complex as claimed in the preceding claim, in which the hydrocarbon-based compound, that is at least a C₁₈ hydrocarbon-based compound, of squalene structure or analog thereof is represented by the radical of formula (I) which follows: 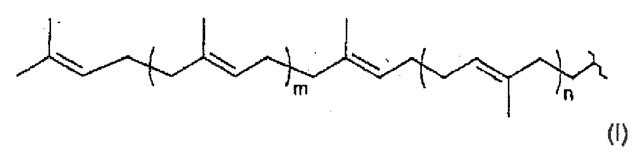 in which:
- m = 1, 2, 3, 4 or 5;
- n = 0, 1, 2, 3, 4 or 5; and
- 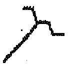 represents the bond to the rest of the complex with the nucleic acid.

3. The complex as claimed in either one of the preceding claims, in which the hydrocarbon-based compound, that is at least a C₁₈ hydrocarbon-based compound, of squalene structure is 1,1',2-trisnorsqualenic acid.

4. The complex as claimed in any one of the preceding claims, in which the molecule of nucleic acid comprising from 10 to 40 nucleotides is an RNA molecule.

5. The complex as claimed in any one of the preceding claims, in which the RNA molecule is an siRNA molecule.

6. The complex as claimed in the preceding claim, in which the siRNA is the RET/PTC1 siRNA.

7. The complex as claimed in the preceding claim, **characterized in that** the RET/PTC1 siRNA has a nucleotide sequence as follows:
5'-CGUUACCAUCGAGGAUCCAdAdA-3' (SEQ ID No: 1).

8. The complex as claimed in any one of the preceding claims, in which the covalent coupling is a covalent bond of ester, ether, thioether, disulfide, phosphate or amide type, preferably disulfide type.

9. The complex as claimed in any one of the preceding claims, represented by general formula (II) which follows : 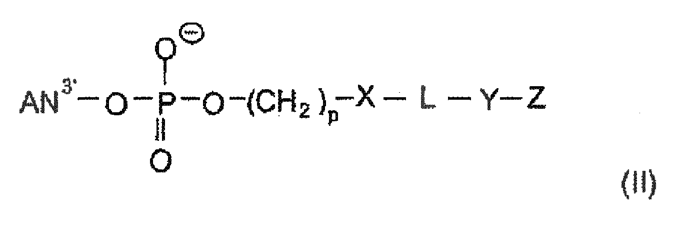
- AN represents a molecule of nucleic acid comprising from 10 to 40 nucleotides, the 3' end of which is linked to the rest of the complex,
- X represents a covalent linkage between the two entities, and more particularly a linkage of ester, ether, thioether, disulfide, phosphate or amide type,
- L represents a linker arm, preferably chosen from saturated alkyl chain units, (poly)amino acid units, polyol units, saccharide units, and polyethylene glycol (polyetheroxide) units of low molecular weight,
- Y represents a covalent linkage, and more particularly a linkage of ester, amide, thioether or phosphate type,
- p represents 0, 1, 2, 3 or 4,
- Z represents a squalenoyl radical or derivative thereof, of formula (I) as defined in claim 2.

10. The complex as claimed in the preceding claim, in which X is a disulfide covalent linkage and Z represents a radical of formula (I) in which m represents 1 and n represents 2.

11. Nanoparticles of a complex as described in any one of claims 1 to 10.

12. The nanoparticles as claimed in the preceding claim, the mean size of which ranges from 30 to 500 nm, in particular from 50 to 250 nm, or even from 100 to 400 nm.

13. A method for preparing nanoparticles as claimed in either one of claims 11 and 12, **characterized in that** it comprises at least:
- the dispersion of a complex as claimed in any one of claims 1 to 10, in at least one organic solvent, at a concentration that is sufficient to obtain, when the resulting mixture is added, with stirring, to an aqueous phase, the instantaneous formation of nanoparticles of said complex in suspension in said aqueous phase, and
- where appropriate, the isolation of said nanoparticles.

14. The nanoparticles as claimed in either of claims 11 and 12, for the use thereof in the treatment and/or prevention of cancers, in particular endocrine cancers, and more particularly capillary thyroid carcinoma.

15. A pharmaceutical composition comprising at least one complex as described in any one of claims 1 to 10 and/or nanoparticles as described in either of claims 11 and 12, in combination with at least one acceptable pharmaceutical vehicle.

## Patentansprüche

1. Komplex, gebildet von mindestens einem Nukleinsäuremolekül, welches 10 bis 40 Nukleotide aufweist und welches mittels einer kovalenten Bindung oder eines Bindungsarms an mindestens eine Kohlenwasserstoffverbindung mit mindestens C₁₈ und mit Squalenstruktur oder ein Analogon davon mit linearer Kohlenwasserstoffstruktur, welches aus Isopreneinheiten gebildet ist, gebunden ist.

2. Komplex gemäß dem vorhergehendem Anspruch, worin die Kohlenwasserstoffverbindung mit mindestens C₁₈ mit Squalenstruktur oder das Analogon davon durch den Rest der folgenden Formel dargestellt wird: 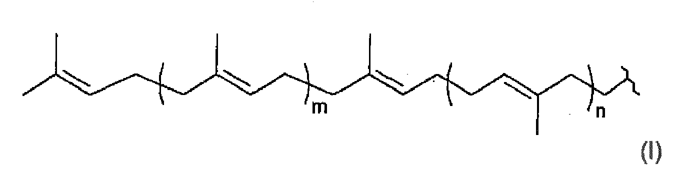 worin:
- m = 1, 2, 3, 4 oder 5;
- n = 0, 1, 2, 3, 4 oder 5; und
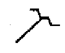 eine Bindung zu dem Rest des Komplexes mit der Nukleinsäure darstellt.

3. Komplex nach einem der vorhergehenden Ansprüche, worin die Kohlenwasserstoffverbindung mit mindestens C₁₈ und Squalenstruktur 1,1',2-Trisnorsqualensäure ist.

4. Komplex nach einem der vorhergehenden Ansprüche, worin das Nukleinsäuremolekül, welches 10 bis 40 Nukleotide umfasst, ein RNA-Molekül ist.

5. Komplex nach einem der vorhergehenden Ansprüche, worin das RNA-Molekül ein siRNA-Molekül ist.

6. Komplex gemäß dem vorhergehenden Anspruch, worin die siRNA die KET/PTC1 siRNA ist.

7. Komplex gemäß dem vorhergehenden Anspruch, gekennzeichnet, dass die RET/PTC1 siRNA eine Nukleotidsequenz wie folgt ist: 5'-CGUUACCAUCGAGGAUCCAdAdA-3' (SEQ ID NO: 1).

8. Komplex nach einem der vorhergehenden Ansprüche, worin die kovalente Bindung eine kovalente Bindung vom Estertyp, Ethertyp, Thioethertyp, Disulfidtyp, Phosphattyp oder Amidtyp ist, bevorzugt vom Disulfidtyp.

9. Komplex nach einem der vorhergehenden Ansprüche, welcher durch die nachfolgende allgemeine Formel (II) dargestellt wird: 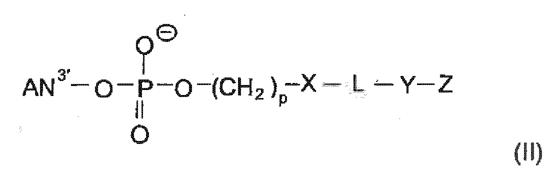
- worin AN ein Nukleinsäuremolekül mit 10 bis 40 Nukleotiden darstellt, wobei das 3'-Ende mit dem Rest des Komplexes verbunden ist,
- X eine kovalente Bindung zwischen den zwei Einheiten darstellt, die insbesondere eine Bindung vom Estertyp, Ethertyp, Thioethertyp, Disulfidtyp, Phosphattyp oder Amidtyp;
- L einen Bindungsarm darstellt, welcher bevorzugt ausgewählt ist aus gesättigten Alkylketteneinheiten, (Poly)aminosäureeinheiten, Polyoleinheiten, Saccharideinheiten und Polyethylenglycoleinheiten (Polyetheroxideinheiten) mit niedrigem Molekulargewicht;
- Y eine kovalente Bindung darstellt und insbesondere eine Bindung vom Estertyp, Amidtyp, Thioethertyp oder Phosphattyp;
- p 0, 1, 2, 3 oder 4 bedeutet, und
- Z einen Squalenoylrest oder ein Derivat davon der Formel (I), wie in Anspruch 2 definiert, darstellt.

10. Komplex gemäß dem vorhergehenden Anspruch, worin X eine kovalente Disulfidbindung ist und Z einen Rest der Formel (I) darstellt, worin m 1 ist und n 2 ist.

11. Nanoteilchen eines Komplexes wie in einem der Ansprüche 1 bis 10 beschrieben.

12. Nanoteilchen gemäß dem vorhergehenden Anspruch, wobei die mittlere Größe von 30 bis 500 nm reicht, insbesondere von 50 bis 250 nm, besonders von 100 bis 400 nm.

13. Verfahren zur Herstellung von Nanoteilchen nach einem der Ansprüche 11 oder 12, **dadurch gekennzeichnet, dass** es mindestens umfasst:
- das Dispergieren eines Komplexes nach einem der Ansprüche 1 bis 10 in mindestens einem organischen Lösungsmittel mit einer ausreichenden Konzentration, damit, wenn die resultierende Mischung unter Rühren zu einer wässerigen Phase gegeben wird, sofort Nanoteilchen des Komplexes in Suspension in der wässerigen Phase gebildet werden, und
- gegebenenfalls Isolieren der Nanoteilchen.

14. Nanoteilchen nach einem der Ansprüche 11 oder 12 zur Verwendung für die Behandlung und/oder Vorbeugung von Krebserkrankungen, insbesondere endokrinen Krebserkrankungen und besonders dem papillären Schildkrüsenkarzinom.

15. Pharmazeutische Zusammensetzung, welche mindestens einen Komplex wie in einem der Ansprüche 1 bis 10 beschrieben und/oder Nanopartikel wie in einem der Ansprüche 11 oder 12 beschrieben in Kombination mit mindestens einem pharmazeutisch akzeptablen Vehikel enthält.
